# EUROPEAN PATENT APPLICATION

(11) **EP 1 452 866 A1**
(43) Date of publication of application: **01.09.2004**
(21) Application number: 02772981.3
(22) Date of filing: 07.10.2002
(51) Int. Cl.: G01N 33/53, C12N 15/00, C12M 1/00, C12Q 1/68, G01N 37/00

(54) **SENSING BOARD**

(30) Priority: 05.10.2001 JP 2001310119; 08.03.2002 JP 2002064350
(71) Applicant: BML, Inc., Tokyo 151-0051 (JP)
(72) Inventor: NOMURA, Sachio, c/o General Laboratory, BML, INC., Kawagoe-shi, Saitama 350-1101 (JP); KENMOTSU, Kazumi, c/o General Laboratory, BML, INC, Kawagoe-shi, Saitama 350-1101 (JP); NAGANO, Makoto, c/o General Laboratory, BML, INC., Kawagoe-shi, Saitama 350-1101 (JP); SAGEHASHI, Yukiko, c/o General Lab., BML, INC., Kawagoe-shi, Saitama 350-1101 (JP); EGASHIRA, Toru, c/o General Laboratory, BML, INC., Kawagoe-shi, Saitama 350-1101 (JP); KONDO, Masatoshi, c/o General Laboratory, BML, INC, Kawagoe-shi, Saitama 350-1101 (JP)
(74) Representative: Bohmann, Armin K., Dr.
(86) International application number: PCT/JP2002/010407
(87) International publication number: WO 2003/031972

(57) **Abstract**

There are provided a) a detection board having numerous wells on a surface thereof, b) a detection set including the detection board and an element serving as well sealing means, and c) a method of using the detection set b), which includes injecting, into each of the wells of the detection board, an element for detecting a liquid phase reaction, sealing the element into the well with the well sealing means, and detecting the liquid phase reaction in the well. Thus, there is provided means for efficiently performing a liquid phase reaction on the board in a manner similar to that of an existing microarray technique.

## Description

### Technical Field

The present invention relates to highly sensitive biological detection means useful, for example, in detection of a specific gene.

### Background Art

Decoding of the primary structure of the human genome is one of the most remarkable achievements in the medical and biological fields in the 20th century.

Results of such genome analysis are envisaged to be employed in a variety of industrial fields and contribute to dramatic technological developments.

The human genome has been found to contain a variety of polymorphic markers, and the vast majority thereof are single nucleotide polymorphisms (SNPs). SNPs are said to account for 80% or more of all polymorphic markers. At present, hopes have been heightened for applications of SNPs in research of disease-related genes and subsequent development of new drugs; i.e., development of drugs based on the human genome.

In addition, detection of SNPs is expected to be useful for, for example, analyzing an individual's physical constitution, and thus will pave the way for individualized medicine.

Currently, in order to improve efficiency of gene analyses, microarrays have been provided. Use of the microarrays enables hybridization or a similar reaction with an extremely large number of nucleotide fragments on a very small chip, leading to elucidation of the function, etc. of specific genes. Thus, through use of such microarrays, hybridization reactions which must be performed for many different combinations of fragments can be performed efficiently, and the amount of a sample to be employed can be considerably reduced as compared with the case where a conventional technique is employed.

In a currently employed microarray technique, a uniform solution prepared from a sample is applied at once onto a chip including a board on which a variety of probes and targets have been immobilized, to thereby perform hybridization. Thus, the microarray technique is efficient analysis means as it enables simultaneous analyses of different sequences in the uniform solution. However, when such an existing microarray technique is employed, difficulty is encountered in performing individual liquid phase reactions by use of a very small amount of a sample.

In general, when a liquid phase reaction is detected, a glass tube is employed for a sample having a volume of up to some ml or thereabouts, and a plastic tube or a plate made up of arrayed plastic tubes (e.g., a 96-well plate, a 384-well plate, or a 1536-well plate) is employed for a volume of some µl or thereabouts. Thus, detection of a liquid phase reaction has been performed for a volume of up to some µl or thereabouts but not yet for a smaller volume (i.e., a volume on the order of nl), for the following reasons. Conventionally performed detection of a liquid phase reaction often employs several to some tens of tubes, and therefore, detection of a liquid phase reaction in a volume on the order of µl or less is considered to be less significant. In addition, technical reasons why detection of a liquid phase reaction in a volume on the order of µl or less is not performed include: 1. change in concentration of a liquid phase due to evaporation becomes significant, whereby difficulty is encountered in performing reaction under optimal conditions; 2. handling of a precisely measured liquid volume is difficult; 3. most plastic materials *per se* exhibit weak autofluorescence, and such autofluorescence becomes background in the case of micro-measurement, interfering in highly sensitive detection; 4. the liquid - container contact area per unit volume of solution increases, so that adverse effects caused by bonding of a component of the solution to the container become considerable; and 5. since the liquid - well contact area per unit volume of solution increases, elution of a trace amount of a component of the container into the liquid phase may adversely affect the liquid phase reaction.

However, as described above, exhaustive analysis of genes of most living organisms (including human) has been considered significant, and accordingly, liquid phase reactions associated with a gene have been provided.

In view of the foregoing, an object of the present invention is to provide means for efficiently performing a liquid phase reaction on a board in a manner similar to that of an existing microarray technique.

### Disclosure of the Invention

In order to achieve the aforementioned object, the present inventors have performed extensive studies, and as a result have found that the aforementioned object can be achieved when numerous microwells are provided on the surface of a board, and detection of a liquid phase reaction is performed for each of the microwells under a closed environment. The present invention has been accomplished on the basis of this finding.

Accordingly, the present invention provides: a) a detection board having numerous wells on a surface thereof (hereinafter may be referred to as "the present detection board"), and b) a detection set comprising the present detection board and an element serving as well sealing means (hereinafter the detection set may be referred to as "the present detection set"). The present invention also provides a method of using the present detection set, which comprises injecting, into each of the wells of the present detection board, an element for detecting a liquid phase reaction, sealing the well containing the element with the well sealing means, and detecting the liquid phase reaction in the well (hereinafter the method may be referred to as "the present use method").

In a most preferred detection mode of the present invention, the liquid phase reaction performed in the present use method is a reaction based on the Invader assay [Third Wave Technologies, Inc. (US)]. In the Invader assay, a nucleotide fragment serving as a template is hybridized with a first nucleotide fragment (1) having a characteristic feature described below in 1, and then hybridized with a second nucleotide fragment (2) having a characteristic feature described below in 2; a nuclease capable of specifically cleaving a locally 3-base overlapping structure formed of these nucleotide fragments on its 3'-side is caused to act on the structure; and a detection portion of the second nucleotide fragment that has been cleaved by the nuclease is detected, whereby a single-base difference in the template nucleotide fragment is detected.
1. First nucleotide fragment: a nucleotide fragment complementary to the template nucleotide fragment, which forms a locally 3-base overlapping structure when the 3'-end base of the first nucleotide fragment interferes in association reaction between a base to be detected and the second nucleotide fragment.
2. second nucleotide fragment: a composite nucleotide fragment including a "complementary portion" which is complementary to the template nucleotide fragment, and a "detection portion" which has a detection element and is not complementary to the template nucleotide fragment, wherein the complementary portion is located on the 3'-side and the detection portion is located on the 5'-side so as to be continuous with the complementary portion, and the base located at the 5'-side end of the "complementary portion" is complementary to the base to be detected.

In the Invader assay, the expression "a nucleotide fragment is complementary to another nucleotide fragment" refers to the case where the nucleotide fragments can form a double-stranded structure through hybridization of the nucleotide fragments, and does not necessarily refer to the case where the nucleotide fragments are 100% complementary to each other. Meanwhile, the expression "a base or a specific base of a nucleotide fragment is complementary to another base" refers to the case where the corresponding bases are complementary to each other.

### Brief Description of the Drawings

Fig. 1 schematically shows the present detection board.
Fig. 2 schematically shows the Invader assay.
Fig. 3 is a photograph showing the results of a preliminary test in relation to the present invention, the test employing a liquid phase reaction based on the Invader assay.
Fig. 4 is photographs showing the results of a first test in relation to the present invention, the test employing a liquid phase reaction based on the Invader assay.
Fig. 5 is a photograph showing the results of a second test in relation to the present invention, the test employing a liquid phase reaction based on the Invader assay.
Fig. 6 is a photograph showing the results of a third test in relation to the present invention, the test employing a liquid phase reaction based on the Invader assay.
Fig. 7 shows data obtained through analysis of the results of the third test employing a liquid phase reaction based on the Invader assay.

### Best Mode for Carrying Out the Invention

Embodiments of the present invention will next be described.

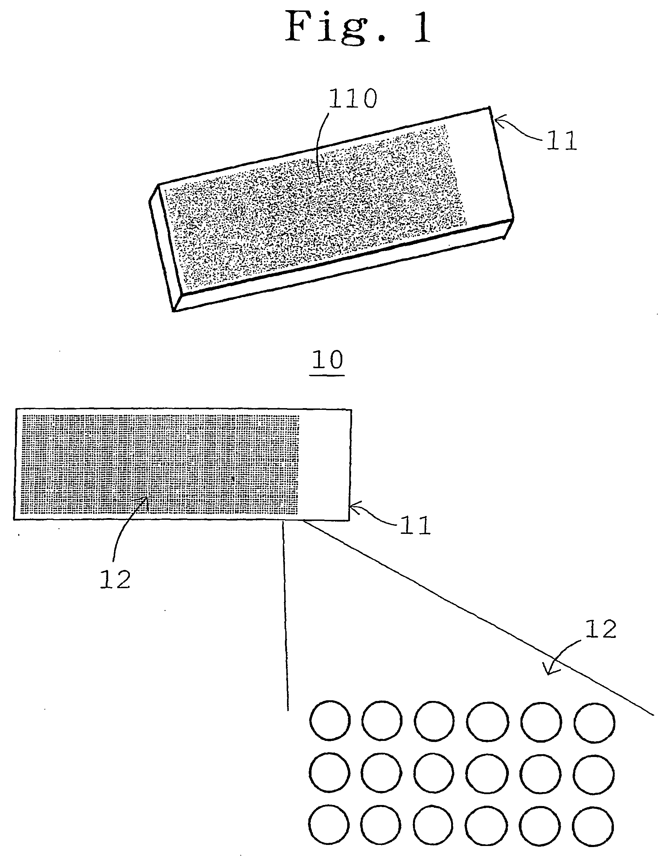

Fig. 1 shows an embodiment of the present detection board. The present detection board 10 is produced by providing numerous (at least two) wells 12 on the surface 110 (only one of the two surfaces) of a baseboard 11.

No particular limitations are imposed on the capacity of each of the wells 12, and the well capacity is appropriately determined in accordance with the volume of a liquid phase required for detection of a liquid phase reaction performed in the well. The capacity of each of the wells 12 must be appropriately greater than the volume of a liquid phase required for detection of a liquid phase reaction. Specifically, the well capacity is preferably about 100 to about 6,800% of the required liquid-phase volume.

In the present detection board 10, detection of a liquid phase reaction the required volume of a liquid phase is preferably on the order of less than µl is performed for each of the wells 12. Therefore, the capacity of each of the wells 12 is preferably 1 µl or less, more preferably about 0.01 µl or less. The preferred lower limit of the capacity of each of the wells 12 should be determined in accordance with the detection sensitivity of a liquid phase reaction and the technique for providing the wells 12.

In the present detection board 10, no particular limitations are imposed on the density of the wells 12 present in a unit area of the board, and the well density is determined in accordance with the size of each of the wells 12, the technique for providing the wells 12, and the technique for detecting a liquid phase reaction. In general, the well density is preferably 1 to 40,000 wells/cm² or thereabouts. In the case where the reaction performed in the present detection board is based on the below-described Invader assay, the well density is particularly preferably 1 to 10,000 wells/cm² or thereabouts. In the case where the Invader assay is performed in a "light treatment" mode [i.e., in the case where the amount of DNAs to be detected is small or the number of single nucleotide polymorphisms (SNPs) to be detected is small; for example, in the case where several hundreds of subjects are subjected to testing and several SNPs are detected], the well density is more preferably one well/cm² or more but less than 400 wells/cm². Meanwhile, in the case where the Invader assay is performed in a "heavy treatment" mode [i.e., in the case where the number of single nucleotide polymorphisms (SNPs) to be detected is large (e.g., several tens of thousands of SNPs or more)], the well density is highly preferably 400 to 10,000 wells/cm² or thereabouts. In the case where the reaction performed on the present detection board is based on a low-density microarray assay (e.g., in the case where expression of several hundreds of genes is to be detected), or a liquid phase reaction other than the low-density Invader assay, such as immune response, radioimmunoassay, or homogeneous assay (e.g., in the case where several species are to be detected in the liquid phase reaction), the well density is particularly preferably one well/cm² or more but less than 400 wells/cm². In the case where the reaction performed on the present detection board is based on a high-density microarray assay (e.g., in the case where expression of several thousands to several tens of thousands of genes is to be detected), or a liquid phase reaction other than the high-density Invader assay (e.g., in the case where several thousands to several tens of thousands of species are to be detected in the liquid phase reaction), the well density is particularly preferably 400 to 40,000 wells/cm², higly preferably 400 to 10,000 wells/cm².

No particular limitations are imposed on the shape of the wells 12, and the wells assume, for example, a semispherical shape, a semispherical-bottomed cylindrical shape, a cylindrical shape, a mortar-like shape, a conical shape, a pyramidal shape, or a rectangular columnar shape. The opening of each of the wells 12 is preferably maintained such that a liquid phase can be readily injected into the well. Specifically, the size of the opening is preferably 0.01 to 0.5 mm (in diameter) or thereabouts.

No particular limitations are imposed on the material of the present detection board 10, so long as the material exhibits sufficient rigidity for practical use. Particularly when the liquid phase reaction detection means is detection means employing fluorescence, preferably, the detection board is formed of a material exhibiting no autofluorescence, from the viewpoint of prevention of generation of background upon detection. In such a case, the present detection board 10 is formed of, for example, glass, ceramic, metal, or plastic.

Examples of thermoplastic resins (plastic materials) include a polymer whose main fragment is formed of substantially solely carbon atoms. Specific examples of such a polymer include olefin-based polymers such as propylene-based polymers (e.g., polypropylene) and 4-methylpentene-1-based polymers; cycloolefin-based polymers such as norbornene-based polymers (e.g., ethylene-norbornene copolymers); acrylic polymers such as methyl methacrylate-based polymers, copolymers of isobornyl methacrylate, and dicyclopentanylmethacrylic copolymers; styrene-based polymers such as amorphous styrene-based polymers, syndiotactic styrene-based polymers, para-t-butylstyrene-based polymers, α-methylstyrene-methyl methacrylate copolymers, and ABS resin; cyclohexyl malate-based polymers; dimethyl itaconate-based polymers; hardened vinyl chloride resin; fluorine-containing polymers (e.g., vinylidene fluoride-based polymers and tetrafluoroethylene-based polymers); and other vinyl-based polymers.

Examples of thermoplastic resins include a polymer whose main fragment contains a hetero atom. Specific examples of such a polymer include polyacetal resin, polycarbonate, polysulfone, aromatic polyester, polyamide, polyurethane, polyphenylene ether, polyphenylene sulfide, polyimide resin, and triacetyl cellulose.

Examples of thermosetting resins include unsaturated polyester, epoxy resin (particularly, alicyclic epoxy resin), three-dimensional hardened polyurethane, unsaturated acrylic resin (including epoxy acrylate resin), melamine resin, three-dimensional styrene resin, three-dimensional silicone resin, and allyl resin (e.g., diallyl phthalate resin or diethylene glycol diallyl carbonate resin).

If desired, the present detection board 10 formed of glass or plastic may be subjected to surface treatment such as silicone treatment or fatty acid treatment by means of a customary method. Particularly, in many cases, the present detection board 10 is preferably subjected to silicone treatment, in order to prevent adsorption of a detection material, a reagent, or the like onto the surface of the board.

Silicone treatment can be carried out by means of a customary method. For example, silicone treatment can be performed through the following procedure: a silicone raw material such as colloidal silica is hydrolyzed by means of the sol-gel method or a similar technique; a hardening catalyst, a solvent, and a leveling agent, and, if desired, a UV absorbing agent or the like are added to the above-hydrolyzed product, to thereby prepare a silicone coating material; and the detection board is coated with the resultant coating material by means of a customary method; for example, preferably, dipping, vapor deposition, spraying, roll coating, flow coating, or spin coating.

The present detection board 10 may be colored, to thereby prevent, for example, autofluorescence of the board in the case of detection employing fluorescence, and adverse effects caused by fluorescence emitted from adjacent wells. When coloring is performed, chromaticity, hue, brightness, etc. may be appropriately determined as desired. In general, the color is preferably black. When a black-color board is produced, a black pigment such as carbon is mixed with the material of the board.

No particular limitations are imposed on the specific size and shape of the present detection board 10, and the size and shape may be determined arbitrarily. However, preferably, the size and shape of the detection board are determined on the basis of generally employed standards for the size and shape of microarray, from the viewpoint of practical use of the detection board. Various microarray analysis apparatuses, microarray-related dispensing apparatuses, etc. are designed in accordance with such standards for microarray, and therefore, the present detection board 10 is preferably designed to have a size and shape which meet with such standards. Specifically, the detection board is preferably designed to have a plate-like shape and have a size nearly equal to that of a glass slide which is generally employed in Japan (i.e., 26 mm in width x 76 mm in length × 1 mm in thickness). Similarly and also preferably, the shape and size of the present detection board 10 may be determined so as to be in agreement with the standards for the shape and size of microarray in regions in which the detection board 10 is to be employed [e.g., US (size: about 1 inch in width × about 3 inches in length) and Europe].

Processes for producing the present detection board 10 are roughly classified into the following two processes: a first process in which wells are provided directly onto a single board, and a second process in which a thin film having numerous through holes is bonded to the surface of a board.

In the first production process, the present detection board 10 having the numerous wells 12 on a surface thereof can be produced by a method of forming microwells, such as, for example, a method in which a thin film which is formed of, for example, vinyl chloride and has numerous through holes corresponding to the wells 12, the thin film serving as a mask, is attached onto the surface of a non-treated board, and microwells are formed on the mask-coated board surface by means of sand blasting (a technique for forming wells on the board surface by hitting microparticles onto the surface at high speed); punching or embossing by use of a die having microirregularities; or machining by use of a microdrill.

When the present detection board 10 is formed of plastic, embossing or punching is preferably employed for forming wells.

In the second production process, a thin film having numerous through holes which are to serve as the wells 12 (the thin film must have a thickness corresponding to the depth of the wells 12, and thus the thin film may be formed of, for example, a plurality of laminated thin films so as to attain such a thickness) is bonded to the surface of a non-treated board. Through this production process, the present detection board 10 having the numerous wells 12 on a surface thereof can be produced.

In the case where the liquid phase reaction detection means is detection means employing fluorescence, the thin film material employed in the second production process is preferably a material which exhibits the lowest possible level of autofluorescence. Preferred examples of such a material include acrylic material and polycarbonate, which exhibit low fluorescence.

In the second production process, no particular limitations are imposed on the method for bonding the thin film to the non-treated board, and the bonding method may employ any known adhesive, such as a reactive adhesive, a UV curing adhesive, an instant adhesive, or a hot-melt adhesive. Of these, a UV curing adhesive, which can be instantaneously cured through irradiation with UV rays at a desired timing, is preferably employed.

The present detection board 10 can be produced through any of the aforementioned production processes.

The present detection set includes the aforementioned present detection board, and an element serving as well sealing means.

Sealing of wells is necessary for preventing change in concentration of liquid-phase contents injected into the wells, which would occur due to evaporation of the contents, so as to attain optimal reaction and correct detection.

Examples of the well sealing means include thin film coating, coating film formed by use of a non-volatile liquid, and coating film formed by use of a material which can be solidified after being applied for sealing wells. Any reaction of the liquid-phase contents which might be induced by such well sealing means is not preferred. Therefore, the well sealing means must be non-reactive to the liquid-phase contents. In order to facilitate detection of a liquid phase reaction after each of the wells are sealed with the well sealing means, in general, to the extent possible the transparency of the sealed portion against the interior of the well is preferably maintained (a portion of the well other than the sealed portion does not necessarily have transparency, since, for example, there could be the case where light reflected in the well is detected). The expression "the transparency of the sealed portion" refers to the case where the sealed portion exhibits a certain level of transparency such that a liquid phase reaction (e.g., fluorescence reaction) in the well can be detected by a detector provided outside the well. The transparency can be determined in accordance with, for example, the type of a liquid phase reaction or the size of the well.

Examples of the element serving as well sealing means which satisfies the aforementioned requirements include a thin film, such as a plastic sole coated with an adhesive; a non-volatile liquid, such as mineral oil or silicone oil; and a material which can be solidified after being applied for sealing wells, such as a UV curing resin.

As described above, detection of the liquid phase reaction in the well can be carried out after injecting an element for detecting a liquid phase reaction into each of the wells of the present detection board, and sealing the wells containing the element with the aforementioned well sealing means (the present use method).

The entirety of the present detection board is placed in a hermetic space (including a chamber which tightly accommodates the entirety of the board), and water or a liquid capable of generating water vapor is provided in the vicinity of the board placed in the space, such that the humidity of the space is maintained in the vicinity of saturated humidity, whereby evaporation of the liquid phase in the wells can be prevented, and effects similar to those obtained through the aforementioned well sealing means can be obtained.

Detection of the liquid phase reaction encompasses detection of the reaction while the liquid phase is sealed into the wells, as well as detection of the reaction after a hole is formed in each of the sealed wells, and then the liquid phase in the well is dried.

No particular limitations are imposed on the liquid phase reaction performed in the present invention. Examples of the liquid phase reaction include protein catalytic reaction such as enzyme reaction, antigen-antibody reaction, interaction between proteins, and specific affinity reaction between substances (including hybridization between nucleotide fragments).

Detection of the liquid phase reaction can be performed through means which is currently employed in microarray techniques. Specifically, for example, the present detection board in which the liquid phase reaction has been performed through the present use method is subjected to analysis by use of a highly sensitive fluorescence scanner, whereby fluorescence in each of the wells of the detection board can be detected. In addition, there can be performed, for example, detection by means of a radioisotope, detection by means of the EIA method, and homogeneous detection by means of AlphaScreen™ [product of PerkinElmer, Inc. (US)].

As described above, in a most preferred mode of the present invention, the liquid phase reaction performed in the present use method is a reaction based on the Invader assay [Third Wave Technologies, Inc. (US)].

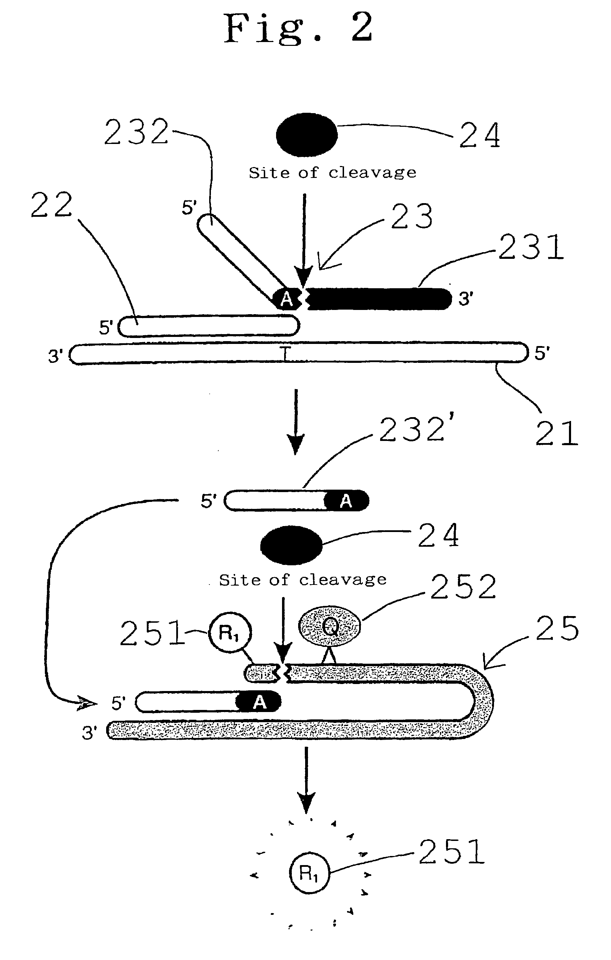

Fig. 2 schematically shows the basic feature of the Invader assay.

As shown in Fig. 2, firstly, a first nucleotide fragment 22 is hybridized with a nucleotide fragment 21 serving as a template (gene polymorphism).

The first nucleotide fragment 22 in which the base [A (adenine) in Fig. 2] complementary to the base to be detected [T (thymine) in Fig. 2] of the template nucleotide fragment 21 is located at the 3'-end is complementary to the template nucleotide fragment 21. (In this case, the base at the 3'-end of the first nucleotide fragment 22 is complementary to the base to be detected. However, even in the case where the 3'-end base is not complementary to the base to be detected, when the 3'-end base interferes in association reaction between the base to be detected and a second nucleotide fragment, a locally 3-base overlapping structure is formed.)

Subsequently, a second nucleotide fragment 23 is hybridized with the locally double-stranded structure formed of the template nucleotide fragment 21 and the first nucleotide fragment 22.

The second nucleotide fragment 23 is a composite nucleotide fragment including a "complementary portion" 231 which is complementary to the template nucleotide fragment 21, and a "detection portion" 232 which has a detection element and is not complementary to the template nucleotide fragment, wherein the portion 231 is located on the 3'-side, and the portion 232 is located on the 5'-side so as to be continuous with the portion 231. The base located at the 5'-side end of the "complementary portion" 231 is a base (A), which is complementary to the base to be detected (T).

This second hybridization forms a locally 3-base overlapping structure including the base to be detected (T) of the template nucleotide fragment 21, the 3'-end base of the first nucleotide fragment 22, and the base (A) located at the 5'-side end of the "complementary portion" 231 of the second nucleotide fragment.

Subsequently, a nuclease 24 having activity to specifically cleave the locally 3-base overlapping structure on its 3'-side is caused to act on the structure, and a detection portion 232' of the second nucleotide fragment 23 which has been cleaved by the nuclease [the 3'-end base of the portion 232' is a base (A), which is complementary to the base to be detected (T)] is detected, whereby the template nucleotide fragment 21 can be detected to be one type of gene polymorphism.

As shown in Fig. 2, when a hairpin-shaped probe (nucleotide fragment) 25 labeled with a fluorescent dye 251 in the vicinity of its 5'-end and with a quencher 252 in the vicinity of its 3'-side is caused to coexist with the aforementioned hybridization system, the aforementioned one type of gene polymorphism can be detected.

A single-stranded portion (on the 3'-side) of the hairpin-shaped probe 25 is designed so as to be complementary to the detection portion 232 of the second nucleotide fragment 23. The base to be detected (T) is one base on the 5'-side which is adjacent to the base located at the 5'-side end of the single-stranded portion. When the detection portion 232' is hybridized with the single-stranded portion of the hairpin-shaped probe 25, at the tip of a double-stranded portion of the probe 25, a locally 3-base overlapping structure is formed of the base (A) located at the 3'-end of the detection portion 232' and the hairpin-shaped probe 25. The nuclease 24 acts on the locally 3-base overlapping structure, and the hairpin-shaped probe 25 is cleaved at a site between a portion labeled with the fluorescent dye 251 and a portion labeled with the quencher 252, whereby the portion labeled with the fluorescent dye 251 is released. Since the thus-released portion is no longer affected by the quencher 252, fluorescence emitted from the released portion can be detected. Through detection of the fluorescence, the template nucleotide fragment 21 can be detected to be the aforementioned one type of gene polymorphism.

Meanwhile, in the case where the base to be detected of the template nucleotide fragment 21 is not the base of one type of gene polymorphism (T) but the base of another type of gene polymorphism [guanine (G)] (i.e., an SNP base), and the base(G) is positively detected, the complementary base of the first nucleotide fragment 22 and the second nucleotide fragment 23 is changed from the above-employed A to C (cytosine), which is complementary to G, and the fluorescent dye 251 and the quencher 252 provided on the hairpin-shaped probe 25 are changed to a fluorescent dye which emits fluorescence differing from the above fluorescence and a quencher corresponding to the fluorescent dye, respectively, whereby the SNP of the template nucleotide fragment 21 can be detected by means of fluorescence emitted from the different fluorescent dye.

When the template nucleotide fragment 21 is a nucleotide fragment including one type of SNP and another type of SNP; i.e., a hetero-type nucleotide fragment, the nucleotide fragment can be positively detected by means of a mixture of the aforementioned two types of fluorescence.

In the above-described embodiment, the detection system employs the hairpin-shaped probe. However, for example, the detection portion 232 can be directly labeled with a fluorescent dye or an isotope, and the thus-labeled detection portion can be directly detected, whereby SNPs, etc. can be detected. In the above-described embodiment, the base of the template nucleotide fragment is positively detected in both the case where the nucleotide fragment has SNPs and the case where the nucleotide fragment does not have SNPs. However, in either of the above cases, negative detection, in which a label such as fluorescence is not detected, can be performed.

In the above-described Invader assay, as reaction proceeds, the nuclease which specifically cleaves the locally 3-base overlapping structure continuously acts in a step in which the "detection portion" of the second nucleotide fragment is cleaved, and in a step in which a portion labeled with a fluorophore is separated from a portion labeled with a quencher (in the case where the hairpin-shaped probe is employed). Therefore, a label employed in the Invader assay, such as fluorescence, is sensitized; i.e., the Invader assay involves a very sensitive liquid phase reaction. When a micro liquid phase reaction like the case of the present invention is detected, employment of the Invader assay is most preferred. The Invader assay is very useful for efficiently detecting SNPs, which are a key to individualized medicine. When the present invention is applied to the Invader assay, SNPs can be detected efficiently and exhaustively. Industrial significance of such efficient and exhaustive detection of SNPs is very high.

A liquid phase reaction in which a nucleic acid, a protein, or the like should be adsorbed onto a well-forming surface is preferably performed on the present detection board, since, in the present detection board, the surface area per unit area of the board is large by virtue of provision of numerous wells, and the density of the nucleic acid or protein immobilized on the detection board can be increased. The immobilization density of the nucleic acid or protein can be considerably increased when the surface of the present detection board on which wells are to be formed is roughened through, for example, grinding by means of sand blasting, or hydrofluoric acid treatment. When the density of the nucleic acid or protein immobilized on the present detection board is increased to thereby increase the volume of such a substance bonded to the board, direct range during detection of the liquid phase reaction can be increased.

In the case of immobilization of a nucleic acid by means of a currently employed microarray technique or a similar technique, numerous pins must be immersed in a nucleic acid solution, and the solution must be applied onto a slide (various arrayers are commercially available). However, in the above method, in many cases, deviation of the pins from the correct positions causes variation in the solution-spotted positions. In such a case, after the resultant signals are detected by, for example a fluorescence scanner, positioning of specific signals (gridding: see, for example, "DNA Microarray Experiment Manual Assuring Provision of Data" (Yodosha Co., Ltd.), page 106) by use of analysis software requires a long period of time. Therefore, the above method is not efficient, and may cause adverse effects on data accuracy.

In the present detection board, the position of the wells is fixed, and thus the aforementioned gridding operation can be considerably efficiently performed. In addition, the detection board does not involve variation in well position, and therefore data accuracy can be maintained.

A liquid-phase employed for detection can be dispensed in the present detection board by use of, for example, an inkjet-type microdispenser synQUAD™ (product of Cartesian (US)). Alternatively, a high-accuracy microdispenser on the order of nano-liter may be employed. Through use of such a microdispenser, dispensing positions can be horizontally and vertically controlled on the order of several micro-meter. Even in the case where the aforementioned pin-type arrayer is employed in the present detection board, when the degree of misalignment of the pin tips fall within a range of the wells on the board, a liquid-phase is spotted in each of the wells, whereby gridding can be performed without causing any problem.

### Examples

The present invention will next be described in more detail by way of Examples.

In the below-described Examples, each of A1·A2, B1·B2, and C1·C2 representing gene polymorphisms is employed as a symbol for distinguishing one type of the gene polymorphism from the other type.

### Test Example 1

A hole (diameter: 6 mm) is formed in a plastic seal having a thickness of about 0.1 µm (a commercially available polycarbonate seal, the same shall apply hereinafter) by use of a puncher, and the hole-formed seal is attached to a glass slide. The Invader assay was performed in the hole provided on the glass slide.

Specifically, the Invader assay employing the following nucleotide fragments was performed in the hole provided on the slide glass. There were employed, as a template nucleotide fragment (hereinafter may be referred to as a "target sequence"), nucleotide fragments partially encoding the drug-metabolizing enzyme CYP4502D6 [two types: A1 and A2, the base in parentheses is the base to be detected] (synthetic DNA): 
5′-AGCTCATAGGGGGATGGG(G)TCACCAGGAAAGCAAAGACACCATGGTGGCTG-
3′ (A1: SEQ ID NO: 1)
 and, 
5′-AGCTCATAGGGGGATGGG(C)TCACCAGGAAAGCAAAGACACCATGGTGGCTG-
3′ (A2: SEQ ID NO: 2).
 There was employed, as a first nucleotide fragment (hereinafter may be referred to as an "invader oligo") (synthetic DNA common between A1 and A2), the following sequence: 5′-GCCACCATGGTGTCTTTGCTTTCCTGGTGAT-3′ (SEQ ID NO: 3). There was employed, as a second nucleotide fragment (hereinafter may be referred to as an "signal probe"), the following sequences (two types: A1 and A2): 5'-CGCGCCGAGGCCCCATCCCCCTATG-3' (A1: SEQ ID NO: 4), and 5'-ATGACGTGGCAGACGCCCATCCCCCTATG-3' (A2: SEQ ID NO: 5). All the invader reagents employed for the Invader assay were reagents prepared by TWT.

The detection portion of the signal probe was detected by use of the aforementioned hairpin-shaped probe (FRET probe). In the case of the A1 target sequence, FAM (green signal) was employed as a fluorophore of the FRET probe for detecting the detection portion of the signal probe, which is cleaved by an enzyme (cleavase) which cleaves a locally 3-base overlapping structure. Meanwhile, in the case of the A2 target sequence, RED (red signal) was employed as a fluorophore of the FRET probe.

A liquid phase employed in the Invader assay (hereinafter may be referred to as an "assay solution") was prepared to contain the target sequence (2.33 fM), the invader oligo (0.07 µM), the signal probe (0.7 µM), and the invader reagent (cleavase: 6.7 ng/µl, MgCl₂: 7.3 mM, MOPS (pH 7.5): 10 mM). The assay solution (0.15 µl) was spotted in the hole provided on the glass slide.

Immediately after spotting of the assay solution, the hole provided on the glass slide was sealed with a plastic seal similar to that described above, the seal having no hole. Thereafter, the thus-sealed glass slide was placed in a microarray reaction container (Hybridization Cassette, product of BM Equipment Co., Ltd.), followed by reaction at 63°C for six hours. Subsequently, the resultant reaction product was subjected to analysis by use of a microarray fluorescence laser scanner (ScanArray 5000, product of Packard (US)). The fluorophores FAM and RED were excited by light of 488 nm and 594 nm, respectively. A 522-nm fluorescence filter and a 614-nm fluorescence filter were employed for FAM and RED, respectively.

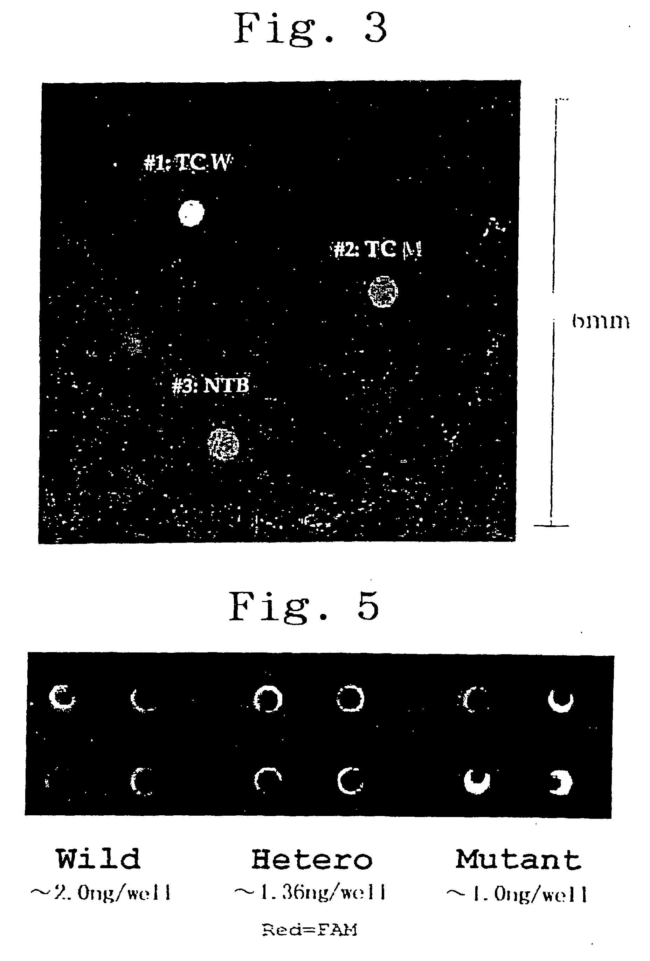

Fig. 3 shows the thus-analyzed image. In Fig. 3, TCW represents the spot corresponding to the detection system employing the A1 target sequence. In TCW, development of green color corresponding to the signal of FAM was confirmed. [It is a false color displayed by means of computer software (QuantArray, product of Pakcard (US)), which is employed for the purpose of showing a selected signal in an easy-to-understand manner. The color corresponding to a selected signal is not determined by the type of the signal, and is arbitrarily selected from among colors determined by the computer software. Therefore, a single signal may be represented by different colors. The same shall apply hereinafter in the present Example.] TCM represents the spot corresponding to the system employing the A2 target sequence. In TCM, development of red color corresponding to the signal of RED was confirmed. NTB represents the spot corresponding to a control system containing no target sequence. In NTB, merely fluorescence (i.e., background) was confirmed.

Thus, when the liquid phase was spotted in the well which was provided on the glass slide by means of a thin film, and the liquid phase reaction was performed in the well after the well was sealed with a transparent thin film (i.e., an element serving as well sealing means), the Invader assay reaction (liquid phase reaction) proceeded normally, and the fluorescence reaction of interest in the Invader assay was positively detected. That is, in circumstance according to a well of the present detection board, the invader oligo and the signal probe were found to be correctly and efficiently hybridized with the target sequence; i.e., hybridization between the nucleic acids proceeded normally, and the locally 3-base overlapping structure was found to be correctly and efficiently cleaved at its 3'-side by means of the cleavase; i.e., the enzyme reaction proceeded normally. The results imply that detection of various liquid phase reactions can be performed by use of the present detection set, while eliminating adverse effects caused by, for example, evaporation of a solvent, which would otherwise occur in a microreaction system.

### Test Example 2

In this Test Example, the Invader assay was performed by use of, instead of synthetic DNA, more diverse human genomic DNA as a target sequence in the present detection set.

The aforementioned plastic seals were laminated and bonded with one another to provide a four-ply seal, and a plurality of through holes (diameter: 4 mm) were formed in the resultant thick plastic seal by use of a puncher such that the through holes were located at intervals of about 5 mm. The seal having the through holes was attached onto a glass slide similar to that employed in Test Example 1 in a manner similar to that of Test Example 1, to thereby prepare the present detection board having a plurality of wells on its surface.

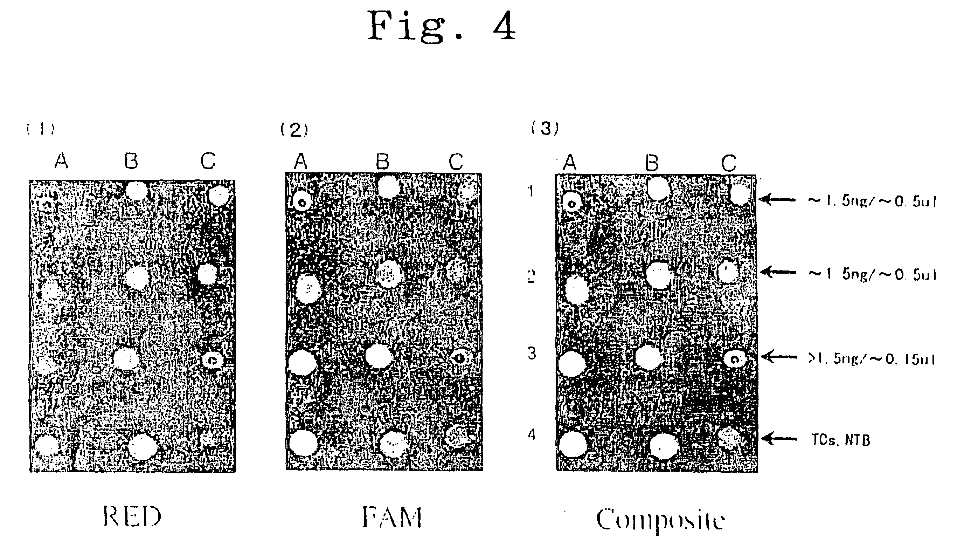

An Invader assay solution was prepared in the same compositions as those in Test Example 1, except that the target sequence was changed to human genomic DNA. In a manner similar to that of Test Example 1, the Invader assay was performed in the below-described test system (the Invader assay solution (about 0.15 µl) was spotted in each of the wells). The results of the Invader assay employing the human genomic DNA are shown in Fig. 4.

Fig. 4 shows the results of the Invader assay employing the human genomic DNA as the target sequence. The amount of the genomic DNA per spot is about 1.5 ng (0.37 zmol) in rows 1 and 2, and the respective rows correspond to genomic DNAs derived from different humans (column A corresponds to human genomic DNA having an A1 homo drug-metabolizing enzyme (CYP4502D6) gene; column B corresponds to human genomic DNA having a hetero drug-metabolizing enzyme (CYP4502D6) gene; and column C corresponds to human genomic DNA having an A2 homo drug-metabolizing enzyme (CYP4502D6) gene). The spots in row 3 correspond to the Invader assay solution containing the same human genomic DNA (i.e., the target sequence) as that in row 1, but the genomic DNA contents of the respective spots differ from one another (3-A: 10 ng, 3-B: 9.5 ng, 3-C: 14.7 ng). Row 4 corresponds to the control group. The spot at 4-A corresponds to the Invader assay solution containing the A1 target sequence (0.35 zmol), which is employed in Test Example 1; the spot at 4-B corresponds to the Invader assay solution containing the A2 target sequence (0.35 zmol), which is employed in Test Example 1; and the spot at 4-C corresponds to the Invader assay solution containing no target sequence.

Fig. 4(1) shows the results for the case where merely RED was caused to emit fluorescence. In the A2 homo group (column C: rows 1 through 3) and the A1/A2 heterozygote group (hereinafter will be referred to as "the hetero group" unless otherwise specified) (column B: rows 1 through 3), emission of red fluorescence was observed. In contrast, in the A1 homo group (column A: rows 1 through 3), virtually no emission of red fluorescence was observed.

Fig. 4(2) shows the results for the case where merely FAM was caused to emit fluorescence. In the A1 homo group (column A: rows 1 through 3) and the hetero group (column B: rows 1 through 3), emission of green fluorescence was observed. In contrast, in the A2 homo group (column C: rows 1 through 3), virtually no emission of green fluorescence was observed.

Fig. 4(3) shows the results for the case where both RED and FAM were caused to emit fluorescence. In the A1 homo group (column A: rows 1 through 3), significant emission of green fluorescence (i.e., fluorescence of FAM) was observed, and in the A2 homo group (column C: rows 1 through 3), significant emission of red fluorescence (i.e., fluorescence of RED) was observed. In addition, in the hetero group (column B: rows 1 through 3), emission of yellow to orange fluorescence based on fluorescence of both FAM and RED was observed. In Figs. 4(1) and 4(2), the homo group and the hetero group are difficult to discriminate from each other. In contrast, in Fig. 4 (3), the homo group and the hetero group are clearly discriminated from each other.

### Test Example 3

In this Test Example, the Invader assay was performed in the present detection set which had been prepared by providing wells more densely on a glass slide by use of human genomic DNA as a target sequence.

Wells were provided more densely on a glass slide (76 mm in length × 26 mm in width × 1 mm in thickness) by means of the aforementioned sand blasting. Specifically, wells (diameter: 0.5 mm, capacity: about 0.1 µl) (32 columns × 100 rows, total: 3,200 wells) were provided on one glass slide such that the distance between the centers of adjacent wells was 0.7 mm, and the resultant slide was coated with a very thin silicone film by means of vapor deposition, to thereby prepare the present detection board.

An Invader assay solution was prepared from a target sequence contained in the human genomic DNA having the below-described sequence, the sequence being a human genomic DNA [cholesteryl ester transfer protein: CETP] gene; an invader oligo having the below-described sequence; and a signal probe having the below-described sequence.

Target sequence (contained in the human genomic DNA)
5′-TGGCTCAGATCTGAACCCTAACT(C)GAACCCCAGTGATTCTGGGTCGCAGACAAAC
AC-3′ (B1: SEQ ID NO: 6)
5′-TGGCTCAGATCTGAACCCTAACT(T)GAACCCCAGTGATTCTGGGTCGCAGACAAAC
AC-3′ (B2: SEQ ID NO: 7)

Invader oligo (synthetic DNA)
5′-GTTTGTCTGCGACCCAGAATCACTGGGGTTCT-3′ (common between B1 and
B2: SEQ ID NO: 8)

Signal probe (synthetic DNA)
5'-ATGACGTGGCAGACGAGTTAGGGTTCAGATCTGA-3' (B1: SEQ ID NO: 9)
5'-CGCGCCGAGGAAGTTAGGGTTCAGATCTGA-3' (B2: SEQ ID NO: 10)

The detection portion of the signal probe was detected by use of the aforementioned hairpin-shaped probe (FRET probe). In the case of the B1 target sequence, FAM (red signal) was employed as a fluorophore of the FRET probe for detecting the detection portion of the signal probe, which is cleaved by an enzyme (cleavase) which cleaves a locally 3-base overlapping structure. Meanwhile, in the case of the B2 target sequence, RED (green signal) was employed as a fluorophore of the FRET probe.

Firstly, the human genomic DNA (target sequence) was spotted into the wells (the B1 homo sequence (about 2.0 ng), the hetero sequence (about 1.36 ng), or the B2 homo sequence (about 1.0 ng) was spotted into the corresponding wells in accordance with the experimental design), followed by heat denaturation at 95°C for five minutes. Subsequently, an assay solution [containing the invader oligo (0.07 µM), the signal probe (0.7 µM), and an invader reagent (cleavase: 6.7 ng/µl, MgCl₂: 7.3 mM, MOPS (pH 7.5): 10 mM)] (about 0.1 µl) was spotted into each of the wells into which the heat-denatured human genomic DNA had been spotted.

Immediately after spotting of the assay solution, the wells provided on the glass slide were sealed with a plastic seal having no hole and made of the same material as that employed in Test Example 1. Thereafter, the thus-sealed glass slide was placed in a microarray reaction container (Hybridization Cassette, product of BM Equipment Co., Ltd.), followed by reaction at 63°C for six hours. Subsequently, the resultant reaction product was subjected to analysis by use of a microarray fluorescence laser scanner (ScanArray 5000, product of Packard (US)). The fluorophores FAM and RED were excited by light of 488 nm and 594 nm, respectively. A 522-nm fluorescence filter and a 614-nm fluorescence filter were employed for FAM and RED, respectively.

Spotting of the human genomic DNA and the assay solution was performed by use of micropipette tips.

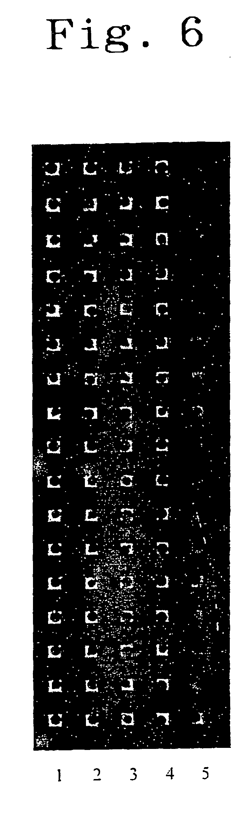

Fig. 5 shows the results of the Invader assay employing the human genomic DNA (fluorescence of the fluorophores in the wells).

In Fig. 5, the left group consisting of four colored spots; i.e., "Wild" group, corresponds to the group employing the CETP B1 homo human genomic DNA as a target sequence. In each of the spots, red fluorescence of FAM is clearly observed. The results show that the present test system can detect the CETP B1 homo human genomic DNA.

The right group consisting of four colored spots; i.e., "Mutant" group, corresponds to the group employing the CETP B2 homo human genomic DNA as a target sequence. In each of the spots, green fluorescence of RED is clearly observed. The results show that the present test system can detect the CETP B2 homo human genomic DNA.

The center group consisting of four colored spots; i.e., "Hetero" group, corresponds to the group employing the CETP heterozygote human genomic DNA as a target sequence. In each of the spots, orange fluorescence; i.e., fluorescence of color between red (corresponding to fluorescence of FAM) and green (corresponding to fluorescence of RED), is clearly observed. The results show that the present test system can also detect the CETP hetero human genomic DNA.

In Test Example 3, after having spotted the assay solution, the above-described test was performed while effecting the following modifications: instead of sealing the board with a plastic seal, 40 µl water was sealed into a microarray reaction container (Hybridization Cassette, product of BM Equipment Co., Ltd.), followed by reaction at 63°C for six hours. As a result, similar to the case of the above-described test, the B1 homo CETP gene, the B2 home CETP gene, and the hetero CETP gene, which are contained in the human genomic DNA, were detected distinctively from one another. In contrast, when 5 µl water was sealed as in usual cases (like the above case where the plastic film is sealed) without sealing with a plastic seal, the liquid phase in each of the wells was evaporated, and the CETP genes failed to be detected.

The results of Test Examples 2 and 3 reveal that when, for example, a human genomic DNA sample is spotted into numerous wells provided on the present detection board such that a trace amount of the sample is spotted into each of the wells, and the thus-spotted sample is subjected to analysis by means of the Invader assay designed for detecting different target bases, the SNPs of the subject from whom the DNA sample was obtained can be exhaustively detected.

### Test Example 4

In this Test Example, the Invader assay was performed with the present detection set which had been prepared by providing wells more densely on a chip formed of carbon-containing polycarbonate by use of the human genomic DNA as a target sequence.

By means of injection molding, wells were provided more densely on a chip formed of polycarbonate containing 1% carbon (size of the chip: 76 mm in length × 27 mm in width × 1.6 mm in thickness). Specifically, there were provided, on the chip, 5,040 (120 × 42) tapered rectangular columnar wells, each having an square opening (0.45 mm × 0.45 mm), a depth of 0.65 mm, a square bottom (0.05 mm × 0.05 mm), and a capacity of 49.3 nl, to thereby prepare the present detection board.

An Invader assay solution was prepared from a target sequence contained in the human genomic DNA having the below-described sequence, the sequence being a human genomic DNA [ATP-binding cassette, sub-family A DNA] gene; an invader oligo having the below-described sequence; and a signal probe having the below-described sequence.

Target sequence (contained in the human genomic DNA)

5′-CATTACCCAGAGGACTGTC(C)GCCTTCCCCTCACCCCAGCCTAGGCC-3′ (C1:
SEQ ID NO: 11)
5′-CATTACCCAGAGGACTGTC(T)GCCTTCCCCTCACCCCAGCCTAGGCC-3′ (C2:
SEQ ID NO: 12)

Invader oligo (synthetic DNA)
5′-CCTAGGCTGGGTGAGGGAAGGCT-3′ (common between C1 and C2:
SEQ ID NO: 13)

Signal probe (synthetic DNA)
5′-ACGGACGCGGAGGGACAGTCCTCTGGGV-3′ [C1: SEQ ID NO: 14 (V:
hexanediol group)]
5′-CGCGCCGAGGAGACAGTCCTCTGGGTV-3′ [C2: SEQ ID NO: 15 (V:
hexanediol group)]

An invader reagent and the sample were dispensed into each of the wells of the chip by use of PS4500 system (Cartesian (US)). Specifically, the reagent and the sample (total volume: 45 nl) were dispensed in each of the wells by use of the system.

The DNA content of each of the wells is as follows.
1) (dispensed into wells of column 1 shown in Fig. 6)
   Target control for FAM (C1): 0.045 zmol/well
2) (dispensed into wells of column 2 shown in Fig. 6)
   Target control for FAM (C2): 0.045 zmol/well
3) (dispensed into wells of column 3 shown in Fig. 6)
   Genomic DNA(G1) <homo for RED>: 0.9 ng/well
4) (dispensed into wells of column 4 shown in Fig. 6)
   Genomic DNA(G1) <hetero for>: 0.9 ng/well
5) (dispensed into wells of column 5 shown in Fig. 6)
   Non target blank (Yeast tRNA): 0.5 ng/well

The enzyme Cleavase XI (TWT (US)) (0.33 ng/well) was employed in the invader reagent. Other components of the reaction solution are as follows: MgCl₂ (15 mM), MOPS (pH 7.5) (10 mM), the invader oligo (0.07 µM), the signal probe (C1) (0.7 µM), the signal probe (C2) (0.7 µM), and Cy5 (0.37 µM).

The aforementioned DNAs 1) through 5) were added to the respective well columns shown in Fig. 6, and then air-dried. Thereafter, the invader reagent was dispensed into each of the wells. Among the aforementioned DNAs, the Genomic DNAs were heat-denatured before being employed. During the course of dispensing of the invader reagent, the humidity was maintained at 100% in order to prevent the solution from being dried.

After completion of dispensing of the invader reagent, a plastic film (may be a commercially available film such as Optical Adhesive Covers (product of Applied Biosystems)) was attached to the surface of the wells, and reaction was performed at 63°C for three hours while preventing drying of the solution.

After completion of reaction, fluorescence of the fluorescent dyes was detected under the following conditions.
a) FAM: laser excitation wavelength 488 nm,
   fluorescence filter 522 nm
b) RED: laser excitation wavelength 543 nm,
   fluorescence filter 592 nm
c) Cy5: laser excitation wavelength 633 nm,
   fluorescence filter 670 nm

After the resultant fluorescence signals were detected, the FAM signals and the RED signals were colored in green and red, respectively, by use of the software "QuantArray" accompanying ScanArray, to thereby produce an image (Fig. 6).

As is clear from Fig. 6, the control for FAM (green) (column 1) and the control for RED (red) (column 2) are correctly recognized. The wells corresponding to the genomic DNA homozygous for RED (G1) assume a red color (column 3), and the wells corresponding to the genomic DNA heterozygous for RED (G2) assume a yellow color; i.e., a color between green and red (column 4).

Thus, a color corresponding to the DNA sample, which had been assumed on the basis of the nature of the DNA sample, was observed by performing the Invader assay by use of the present detection board.

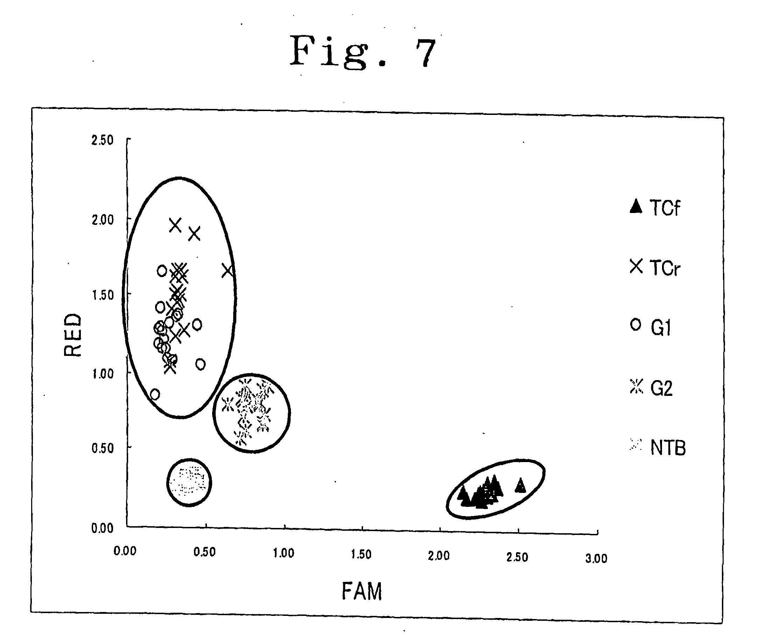

Fig. 7 is a graph showing data obtained by quantifying the image of Fig. 6. In Fig. 7, the horizontal axis corresponds to the fluorescence intensity of FAM, and the vertical axis corresponds to the fluorescence intensity of RED. For correction of sample volume, each of the FAM and RED fluorescence intensities is divided by the intensity of the Cy5 signal. As shown in Fig. 7, the groups of 1) the control for FAM, 2) the control for RED and the genomic DNA homozygous for RED, 3) the genomic DNA heterozygous for RED, and 4) the negative control are clearly separated from one another. Therefore, the data can be employed for determination of gene polymorphisms.

The above results show that gene polymorphism analysis of human genomic DNA (on the order of ng or less) can be reliably and efficiently performed by means of combination of the present detection board and the Invader assay, without employment of a gene amplification technique such as PCR.

### Industrial Applicability

The present invention provides means for efficiently performing a liquid phase reaction on a board in a manner similar to that of an existing microarray technique.

## Claims

1. A detection board having numerous wells on a surface thereof.

2. The detection board according to claim 1, which comprises a thin film having numerous through holes, the film being bonded to the surface of the board.

3. The detection board according to claim 1 or 2, wherein the wells satisfy the following requirements 1 and 2:
1. the capacity of each of the wells: 0.001 to 1 µl, and
2. the density of the wells present on the board: 1 to 40,000 wells/cm².

4. A detection set comprising a detection board as recited in any of claims 1 through 3, and an element serving as well sealing means.

5. The detection set according to claim 4, wherein the element serving as well sealing means is a thin film.

6. The detection set according to claim 4, wherein the element serving as well sealing means is a non-volatile liquid.

7. The detection set according to claim 4, wherein the element serving as well sealing means is a material which can be solidified after being applied for sealing a well.

8. The detection set according to any of claims 4 through 7, wherein the element serving as well sealing means is an element capable of providing a well-sealing portion exhibiting transparency.

9. A method of using a detection set as recited in any of claims 4 through 8, which comprises injecting, into each of the wells of the detection board, an element for detecting a liquid phase reaction, sealing the well containing the element with the well sealing means, and detecting the liquid phase reaction in the well.

10. The method according to claim 9, wherein the liquid phase reaction is a reaction based on a detection method in which a nucleotide fragment serving as a template is hybridized with a first nucleotide fragment (1) having a characteristic feature described below in 1, and then hybridized with a second nucleotide fragment (2) having a characteristic feature described below in 2; a nuclease capable of specifically cleaving a locally 3-base overlapping structure formed of these nucleotide fragments on its 3'-side is caused to act on the structure; and a detection portion of the second nucleotide fragment that has been cleaved by the nuclease is detected, whereby a single-base difference in the template nucleotide fragment is detected:
1. first nucleotide fragment: a nucleotide fragment complementary to the template nucleotide fragment, which forms a locally 3-base overlapping structure when the 3'-end base of the first nucleotide fragment interferes in association reaction between a base to be detected and the second nucleotide fragment; and
2. second nucleotide fragment: a composite nucleotide fragment including a "complementary portion" which is complementary to the template nucleotide fragment, and a "detection portion" which has a detection element and is not complementary to the template nucleotide fragment, wherein the complementary portion is located on the 3'-side and the detection portion is located on the 5'-side so as to be continuous with the complementary portion, and the base located at the 5'-side end of the "complementary portion" is complementary to the base to be detected.


SEQUENCE LISTING

<110> BML, INC

<120> Plate for Detection

<130> B 10043 EP

<140>
<141>

<150> JP P2001-310119
<151> 2001-10-05

<150> JP P2002-064350
<151> 2002-03-08

<160> 15

<170> PatentIn Ver. 2.1

<210> 1
<211> 51
<212> DNA
<213> Artificial Sequence

<220>
<223> Description of Artificial Sequence:Target Sequence

<400> 1
agctcatagg gggatggggt caccaggaaa gcaaagacac catggtggct g 51

<210> 2
<211> 51
<212> DNA
<213> Artificial Sequence

<220>
<223> Description of Artificial Sequence:Target Sequence

<400> 2
agctcatagg gggatgggct caccaggaaa gcaaagacac catggtggct g 51

<210> 3
<211> 31
<212> DNA
<213> Artificial Sequence

<220>
<223> Description of Artificial Sequence: Invader Oligo

<400> 3
gccaccatgg tgtctttgct ttcctggtga t 31

<210> 4
<211> 25
<212> DNA
<213> Artificial Sequence

<220>

<223> Description of Artificial Sequence: Signal Probe

<400> 4
cgcgccgagg ccccatcccc ctatg                                          25


<210> 5
<211> 29
<212> DNA
<213> Artificial Sequence

<220>
<223> Description of Artificial Sequence: Signal Probe

<400> 5
   atgacgtggc agacgcccat ccccctatg                                     29


<210> 6
<211> 57
<212> DNA
<213> Hominidae

<400> 6
tggctcagat ctgaacccta actcgaaccc cagtgattct gggtcgcaga caaacac    57


<210> 7
<211> 57
<212> DNA
<213> Hominidae

<400> 7
tggctcagat ctgaacccta acttgaaccc cagtgattct gggtcgcaga caaacac


<210> 8
<211> 32
<212> DNA
<213> Artificial Sequence

<220>
<223> Description of Artificial Sequence: Invader Oligo

<400> 8
gtttgtctgc gacccagaat cactggggtt ct


<210> 9
<211> 34
<212> DNA
<213> Artificial Sequence

<220>
<223> Description of Artificial Sequence: Signal Probe

<400> 9
atgacgtggc agacgagtta gggttcagat ctga


<210> 10
<211> 30
<212> DNA

<213> Artificial Sequence

<220>
<223> Description of Artificial Sequence: Signal Probe

<400> 10
cgcgccgagg aagttagggt tcagatctga
[?]@
<210> 11
<211> 46
<212> DNA
<213> Hominidae

<400> 11
cattacccag aggactgtcc gccttcccct caccccagcc taggcc


<210> 12
<211> 46
<212> DNA
<213> Hominidae

<400> 12
cattacccag aggactgtct gccttcccct caccccagcc taggcc


<210> 13
<211> 25
<212> DNA
<213> Artificial Sequence

<220>
<223> Description of Artificial Sequence: Invader Oligo

<400> 13
cctaggctgg ggtgagggga aggct


<210> 14
<211> 27
<212> DNA
<213> Artificial Sequence

<220>
<223> Description of Artificial Sequence: Signal Probe

<400> 14
acggacgcgg agggacagtc ctctggg


<210> 15
<211> 26
<212> DNA
<213> Artificial Sequence

<220>
<223> Description of Artificial Sequence: Signal Probe

<400> 15
cgcgccgagg agacagtcct ctgggt